# EUROPÄISCHE PATENTSCHRIFT

(11) **EP 2 371 308 B1**
(45) Veröffentlichungstag und Bekanntmachung des Hinweises auf die Patenterteilung: **11.05.2016**
(21) Anmeldenummer: 11160103.5
(22) Anmeldetag: 29.03.2011
(51) Int. Cl.: A61B 17/29

(54) **Medizinisches Instrument**
Medical instrument
Instrument médical

(30) Priorität: 01.04.2010 DE 102010013916
(43) Veröffentlichungstag der Anmeldung: 05.10.2011
(73) Patentinhaber: Karl Storz GmbH & Co. KG, 78532 Tuttlingen (DE)
(72) Erfinder: Besse, Régis, 78280, Guyancourt (FR); Thouément, Yann, 78690, Les Essarts le roi (FR)

(56) Entgegenhaltungen:
- EP-A1- 0 594 004
- WO-A2-00/69364
- US-A- 5 251 638
- US-A1- 2004 158 233

## Beschreibung

Die Erfindung betrifft ein medizinisches Instrument mit einem hohlen Schaft, an dessen proximalem Ende eine aus mindestens zwei Griffteilen bestehende Handhabe und an dessen distalem Ende ein Werkzeug angeordnet ist, wobei das Werkzeug mittels der Handhabe über ein in dem Schaft axial verschiebbar gelagertes Betätigungselement betätigbar ist.

Ein gattungsgemäßes medizinisches Instrument ist beispielsweise aus der US 5 501 698 A bekannt. Bei diesem bekannten medizinischen Instrument sind die Handhabe und das Betätigungselement über verschwenkbar an den Griffteilen sowie am Betätigungselement gelagerte Anlenkhebel miteinander gekoppelt. Die Verwendung der zwischengeschalteten verschwenkbaren Anlenkhebel stellt aufgrund der vielen miteinander zu koppelnden Bauteile einen hohen Montageaufwand dar.

Die DE 693 25 625 T2 beschreibt ein Instrument zum Anbringen einer chirurgischen Klemme in Körpergewebe beziehungsweise zum Anbringen einer chirurgischen Klemme zur Verbindung von Blutgefäßen. Das gattungsgemäße Instrument verfügt über einen Betätigungsmechanismus mit einer Nockenschlitz-Stift-Verbindung. Die Verformungsnocke, in welcher die Nockenschlitze angeordnet sind, ist flächig ausgebildet.

Aus der EP-A-0594004 ist ein chirurgisches Klammergerät mit einem kreuzförmigen Mitnehmer bekannt.

Davon ausgehend liegt der Erfindung die Aufgabe zugrunde, ein medizinisches Instrument der eingangs genannten Art zu schaffen, das bei einfachem Aufbau eine direkte und präzise Steuerung des Werkzeugs gewährleistet.

Die Lösung dieser Aufgabenstellung ist in einem medizinischen Instrument mit den Merkmalen des Hauptanspruches gegeben. Vorteilhafte Weiterbildungen der Erfindung sind Gegenstand der Unteransprüche. Es ist dabei besonders wichtig, dass das Betätigungselement ein Zug-/Druckelement aufweist, das als axialverschiebbar im Schaft gelagertes kreuzförmiges Element mit einem in Instrumentenlängsachse ausgerichteten Grundkörper und wenigstens zwei nach außen abstehenden, stabförmigen Armen ausgebildet ist und dass die Griffteile der Handhabe und die Arme des Zug-/ Druckelements über eine Zapfen-Schlitz-Steuerung in Wirkverbindung miteinander stehen.

Unter dem genannten kreuzförmigen Element versteht sich ein Grundkörper mit wenigstens zwei nach außen abstehenden Armen. Die Arme können sowohl rechtwinklig als auch in einem stumpfen oder spitzen Winkel zum Grundkörper angeordnet sein, so dass das kreuzförmige Element T-förmig Y-Förmig, oder Pfeilförmig mit beidseitigem Überstand des Grundkörpers ausgebildet sein kann. In einer bevorzugten Ausführungsform sind die abstehenden Arme insbesondere spiegelsymmetrisch zum Grundkörper angeordnet und treffen sich in einem gemeinsamen Punkt auf der Längsachse des Grundkörpers. Dies ist vor allem in statischer und konstruktiver Hinsicht von großem Vorteil. Aber auch der Herstellungsprozess des Grundkörpers kann durch eine spiegelsymmetrische Anordnung vereinfacht werden.

Eine stabförmige Ausbildung der von dem Grundkörper abstehenden Arme ermöglicht eine platzsparende wie auch stabile Lösung. Unter "stabförmig" wird in diesem Zusammenhang ein Element verstanden, dessen Querschnittsgestalt insbesondere rund als auch eckig ausgestaltet sein kann und dessen Verhältnis zwischen Länge des Stabes und Querschnittsabmessung des Stabes mindestens 3:2, insbesondere 3:1 oder mehr ist.

Die Ausbildung der Kopplung zwischen der Handhabe und dem im Schaft gelagerten Betätigungselement als Zapfen-Schlitz-Steuerung bietet die Möglichkeit einer direkten Umsetzung der Schwenkbewegung der Griffteile der Handhabe in die Axialbewegung des Betätigungselements ohne das Zwischenschalten weiterer Umlenkelemente.

Zur Ausbildung der Zapfen-Schlitz-Steuerung wird erfindungsgemäß vorgeschlagen, dass die Zapfen-Schlitz-Steuerung aus direkt oder indirekt an einem der miteinander zu koppelnden Bauteile, nämlich den Griffteilen oder den Armen, ausgebildeten Führungsbahnen und direkt oder indirekt an dem anderen der miteinander zu koppelnden Bauteilen, nämlich den Armen oder den Griffteilen, ausgebildeten und in den Führungsbahnen gelagerten Steuerzapfen besteht.

Gemäß einer praktischen Ausführungsform der Erfindung sind die Führungsbahnen in den Griffteilen der Handhabe ausgebildet und die Steuerzapfen mit den Armen gekoppelt. Diese Art der Ausbildung der Zapfen-Schlitz-Steuerung stellt eine besonders einfach herzustellende und leicht zu montierende Ausgestaltungsform der Erfindung dar.

Mit einer bevorzugten Ausführungsform der Erfindung wird weiterhin vorgeschlagen, dass das Betätigungselement in Instrumentenlängsachse mehrteilig ausgebildet ist, wobei die einzelnen Teile des Betätigungselements über einen Kopplungsmechanismus miteinander koppelbar sind, um die Montage der Zapfen-Schlitz-Steuerung unabhängig von dem im Schaft gelagerten Betätigungselement ausführen zu können.

Gemäß einer praktischen Ausführungsform der Erfindung ist der Kopplungsmechanismus mit einer Aufnahme Vorrichtung zur Kopplung von Verbindungselementen unterschiedlicher Querschnittsgestalt ausgestaltet. Das Verbindungselement verbindet das Zug-/ Druckelement mit dem Werkzeug am distalen Ende des Betätigungselements und überträgt die axiale Bewegung des Betätigungselements, das insbesondere aus dem Zug-/ Druckelement, dem Kopplungsmechanismus und dem Verbindungselement besteht, auf das Werkzeug. Auf diese Weise wird ein modulares Instrumentenkonzept ermöglicht, da Verbindungselemente mit unterschiedlicher Querschnittsgestalt, insbesondere Querschnittsgröße und Querschnittsform, in der Aufnahmevorrichtung eines einzigen Handgriffes verwendet werden können. Auf diese Weise kann der Anwender mit einem Handgriff zahlreiche Verbindungselemente beispielsweise Zangeneinsätze mit unterschiedlichem Durchmesser und Werkzeugausbildung betätigen und die Anzahl der Instrumente auf dem OP - Tisch kann reduziert werden.

In einer bevorzugten Ausgestaltung weist die Aufnahmevorrichtung des Kopplungsmechanismus ein Verriegelungselement auf, das das proximale Ende eines Verbindungselements, das in seiner Querschnittsgestalt variieren kann, in einer Aufnahme vor ungewollten Lösen sichert. Dadurch ist eine Verschiebbarkeit des Verbindungselements in axialer Richtung durch das Verriegelungselement der Aufnahme unabhängig vom_Zug-/ Druckelement nicht mehr möglich und damit eine verlässliche Kraftübertragung von den Griffteilen über das Betätigungselement mit dem Verbindungselement und dem kreuzförmigen Zug-/ Druckelement auf das Werkzeug gegeben.

Vorteilhafterweise sind die Steuerzapfen an einem das proximale Ende des Betätigungselements bildenden Zug-/Druckelement ausgebildet.

Selbstverständlich ist es alternativ auch möglich, die Steuerzapfen direkt am Betätigungselement anzuordnen.

Gemäß einer praktischen Ausführungsform zur Ausbildung des Zug-/Druckelements wird erfindungsgemäß vorgeschlagen, dass das Zug-/Druckelement als axialverschiebbar im Schaft gelagerte kreuzförmige Stange mit einem in Instrumentenlängsachse ausgerichteten Grundkörper und zwei nach außen abstehenden Armen ausgebildet ist, wobei die Steuerzapfen an den freien Enden der beiden Arme angeordnet sind. Vorteilhafterweise wird vorgeschlagen, dass das kreuzförmige Zug-/Druckelement als T-förmige Stange mit einem in Instrumentenlängsachse ausgerichteten Grundkörper und zwei im Wesentlichen rechtwinklig nach außen abstehenden Armen ausgebildet ist, wobei die Steuerzapfen an den freien Enden der beiden Arme angeordnet sind.

Um das Werkzeug sowie die Griffteile der Handhabe immer wieder automatisch in eine Ausgangsposition zu überführen, wird mit der Erfindung vorgeschlagen, dass das Betätigungselement in distaler Richtung über ein Federelement, vorzugsweise eine Druckfeder, vorgespannt ist.

Schließlich wird mit der Erfindung vorgeschlagen, dass die Steuerzapfen um ihre Längsachse drehbeweglich gelagert sind, beziehungsweise um feststehende Steuerzapfen rotierbare Laufrollen angeordnet sind. Die drehbewegliche Lagerung der Steuerzapfen reduziert den Reibwiderstand beim Verfahren der Steuerzapfen innerhalb der Führungsbahnen.

Weitere Merkmale und Vorteile der Erfindung ergeben sich anhand der zugehörigen Zeichnung, in der ein Ausführungsbeispiel eines erfindungsgemäßen medizinischen Instruments nur beispielhaft dargestellt ist, ohne die Erfindung auf dieses Ausführungsbeispiel zu beschränken. In der Zeichnung zeigt:
- Fig. 1: eine schematische Seitenansicht eines erfindungsgemäßen medizinischen Instruments;
- Fig. 2: eine vergrößerte Ansicht des Details II gemäß Fig. 1;
- Fig. 3: einen Längsschnitt durch das Detail gemäß Fig. 2 und
- Fig. 4: eine Ansicht des Zug-/Druckelements gemäß Fig. 3.
- Fig. 5: eine vergrößerte Ansicht des Details III gemäß Fig. 3

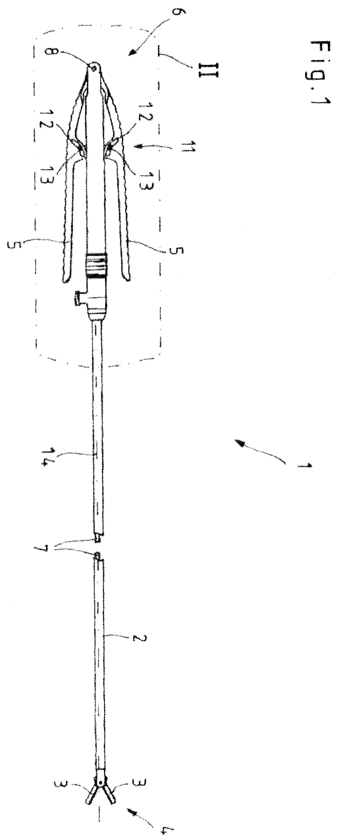

Das in Abbildung Fig. 1 dargestellte medizinische Instrument 1 besteht im wesentlichen aus einem hohlen Schaft 2, an dessen distalem Ende ein aus zwei Maulteilen 3 bestehendes Werkzeug 4 und an dessen proximalem Ende eine aus zwei Griffteilen 5 bestehende Handhabe 6 angeordnet ist. Bei der dargestellten Ausführungsform besteht das Werkzeug 4 aus zwei gegeneinander verschwenkbaren Maulteilen 3. Zum Überführen der Maulteile 3des Werkzeugs 4 von einer geöffneten Ausgangsstellung in eine geschlossene Arbeitsstellung stehen die verschwenkbaren Maulteile 3 des Werkzeugs 4 und die Griffteile 5 der Handhabe 6 über ein in dem hohlen Schaft 2 axial verschiebbar gelagertes Betätigungselement 7 in Wirkverbindung miteinander.

Alternativ zur dargestellten Ausgestaltung des Werkzeugs 4 mit zwei verschwenkbaren Maulteilen 3 ist es auch möglich, das Werkzeug 4 aus einem starren Maulteil 3 und nur einem verschwenkbaren Maulteil 3 bestehend auszubilden. Ebenso ist es möglich, das Werkzeug 4 aus nur einem Werkzeugteil bestehend auszubilden, beispielsweise als Haken oder Spitze, das über die Handhabe 6 bzw. das Betätigungselement 7 vor und zurück bewegbar ist.

Die Griffteile 5 der Handhabe 6 sind bei der dargestellten Ausführungsform um eine gemeinsame Schwenkachse 8 verschwenkbar am proximalen Ende des Schaftes 2 gelagert und weisen mit ihren freien Enden in Richtung des distalen Endes des Schaftes 2.

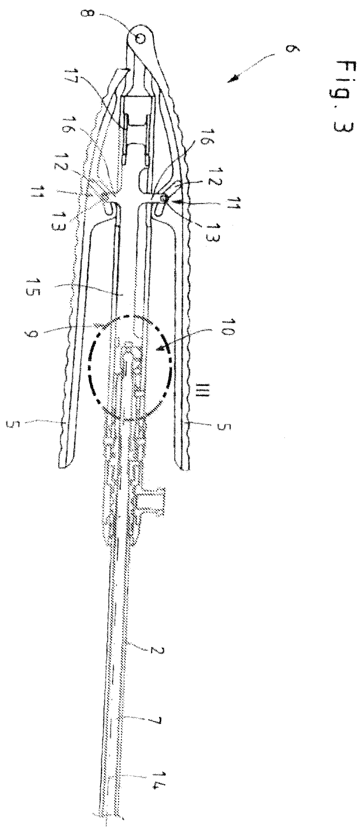

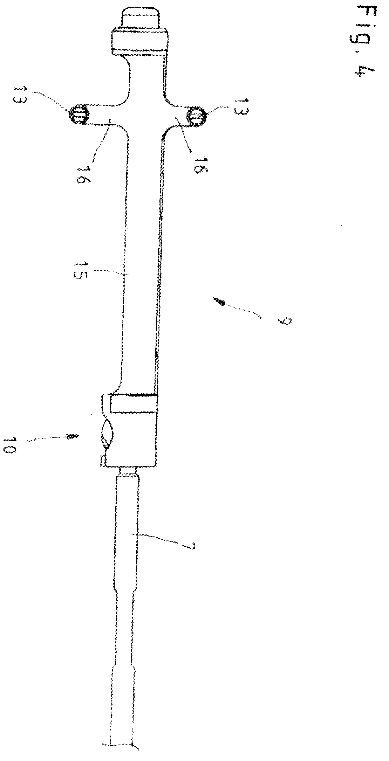

Bei der in den Abbildungen Fig. 3 und 4 dargestellten Ausführungsform ist das Betätigungselement 7 zweiteilig so ausgebildet, dass das proximale Ende des Betätigungselements 7 als separates Zug-/Druckelement 9 ausgebildet, das über einen Kopplungsmechanismus 10 mit distalen Teil des Betätigungselements 7 im Wesentlichen spielfrei koppelbar ist.

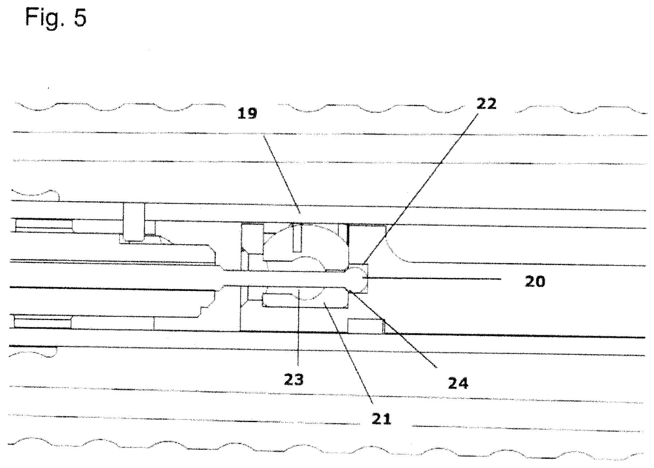

Wie aus Fig. 5 ersichtlich ist, besteht der Kopplungsmechanismus 10 aus einer Aufnahmevorrichtung 19 mit einem Verriegelungselement 21, in welche das Verbindungselement 20 aufgenommen wird. Das Verbindungselement 20 ist an seinem proximalen Ende kugelförmig ausgebildet. Wenn die Griffteile 5 vollständig geöffnet sind, befindet sich das kreuzförmige Element, der Grundkörper 15 und die Arme 16 in distaler Position. Das Verriegelungselement 21 befindet sich hierbei in einer Stellung, welche die Aufnahme eines Verbindungselements 20 ermöglicht. Da das kugelförmige Element am proximalen Ende des Verbindungselements 20 einen kleineren Durchmesser als eine Bohrung 24 im Verriegelungselement 21 aufweist, wird es hierbei durch das Verriegelungselement 21 hindurchgeführt und in der kleineren Aufnahme 22 der beiden Aufnahmen aufgenommen. Werden nun die Griffteile 5 in Richtung des Grundkörpers 15 zusammengedrückt, fährt das kreuzförmige Zug-/ Druckelement 9 in proximale Richtung. Hierbei wird das Verriegelungselement 21 über Steuerstifte (in Fig. 5 nicht ersichtlich) in eine Verriegelungsposition gedreht. Dadurch ist das Verbindungselement 20 außerhalb des Verriegelungselements 21 in der kleinen Aufnahme 22 fest verankert und kann nicht mehr axial verschoben werden. Das kreuzförmige Zug-/ Druckelement 9 ist nun fest über die Aufnahmevorrichtung 19 mit dem Verbindungselement 20 verbunden.

Alternativ kann das kugelförmige Element am proximalen Ende des Verbindungselements 20 auch einen größeren Durchmesser als die Bohrung 24 aufweisen, so dass es nicht durch das Verriegelungselement 21 hindurchgeführt werden kann und in einer großen Aufnahme 23 im Zentrum des Verriegelungselements 20 gehalten wird. Durch ein Betätigen der Griffteile 5, wird das Verriegelungselement 21 auch bei dieser Anwendung über Steuerstifte in eine Verriegelungsposition gedreht, so dass sich das proximale Ende des Verbindungselements 20 nicht mehr aus dem Zentrum des Verriegelungselements 21 lösen kann und das Zug-/ Druckelement fest mit dem Verbindungselement 20 gekoppelt ist.

Damit wird eine Zuordnung des Verbindungselements (20) entsprechend seiner Querschnittsgestalt zu einer entsprechenden Aufnahme sichergestellt.

Alternativ zu der dargestellten zweiteiligen Ausbildung des Betätigungselements 7 mit einem Zug-/ Druckelement 9 und einem Verbindungselement 20 ist es selbstverständlich auch möglich, das Betätigungselement 7 einteilig, oder auch aus mehr als zwei Teilen bestehend auszubilden, wobei bei der mehrteiligen Ausbildung alle Teile des Betätigungselements 7 über entsprechende Kopplungsmechanismen 10 miteinander koppelbar sind.

Der Aufbau des Zug-/Druckelements 9 sowie die Art der Umwandlung der Schwenkbewegung der Griffteile 5 der Handhabe 6 in die ausschließliche Axialbewegung des Betätigungselements 7 ist insbesondere der Abbildung Fig. 3 zu entnehmen.

Wie aus Fig. 3 ersichtlich, stehen die Griffteile 5 der Handhabe 6 und das Betätigungselement 7 über eine Zapfen-Schlitz-Steuerung 11 in Wirkverbindung miteinander. Bei der dargestellten Ausführungsform besteht die Zapfen-Schlitz-Steuerung 11 aus an den Griffteilen 5 der Handhabe 6 ausgebildeten Führungsbahnen 12 und am Betätigungselement 7 ausgebildeten und in den Führungsbahnen 12 gelagerten Steuerzapfen 13. Die Steuerzapfen 13 sind dabei an dem als separates Zug-/Druckelement 9 ausgebildeten proximalen Teil des Betätigungselements 7 angeordnet.

Alternativ zu der dargestellten Ausbildung der Zapfen-Schlitz-Steuerung 11 ist es selbstverständlich auch möglich, die Steuerzapfen 13 an einem einstückigen Betätigungselement 7 anzuordnen oder aber, die Steuerzapfen 13 an den Griffteilen 5 anzuordnen und die korrespondierenden Führungsbahnen am Betätigungselement 7 auszubilden.

Um den Reibwiderstand zwischen den Steuerzapfen 13 und den Führungsbahnen 12 beim Verfahren der Steuerzapfen 13 innerhalb der Führungsbahnen 12 zu reduzieren, sind die Steuerzapfen 13 um ihre Längsachse drehbeweglich gelagert, so dass diese beim Betätigen der Griffteile 5 in den Führungsbahnen 12 abrollen. Alternativ können auch drehbare Rollen auf feststehenden Zapfen angebracht sein.

Das den proximalen Teil des Betätigungselements 7 bildende Zug-/Druckelement 9 ist bei der dargestellten Ausführungsform als axialverschiebbar im Schaft 2 gelagerte T-förmige Stange mit einem in Instrumentenlängsachse 14 ausgerichteten Grundkörper 15 und zwei im Wesentlichen rechtwinklig spiegelsymmetrischen nach außen abstehenden stabförmigen Armen 16 ausgebildet, wobei die Steuerzapfen 13 an den freien Enden der beiden stabförmigen Arme 16 angeordnet sind.

Über die in den Führungsbahnen 12 gelagerten Steuerzapfen 13 der Zapfen-Schlitz-Steuerung 11 wird die Schwenkbewegung der Griffteile 5 der Handhabe 6 in eine reine Axialbewegung des Betätigungselements 7 in Längsrichtung des Schaftes 2 umgewandelt.

Bei der dargestellten Ausführungsform der Zapfen-Schlitz-Steuerung 11 ist die Geometrie der Führungsbahnen 12 so ausgebildet, dass beim Auseinanderziehen der Griffteile 5 der Handhabe 6 das Zug-/Druckelement 9 sowie das mit dem Zug-/Druckelement 9 gekoppelte Betätigungselement 7 in axialer Richtung nach distal verschoben werden und die verschwenkbaren Maulteile 3 des Werkzeugs 4 in die geöffnete Stellung überführen.

Umgekehrt werden beim Zusammendrücken der Griffteile 5 der Handhabe 6 das Zug-/Druckelement 9 sowie das mit dem Zug-/Druckelement 9 gekoppelte Betätigungselement 7 in axialer Richtung nach proximal gezogen, wodurch die verschwenkbaren Maulteile 3 des Werkzeugs 4 in die geschlossene Stellung überführt werden.

Wie weiterhin aus Fig. 3 ersichtlich, ist das Betätigungselement 7 in distaler Richtung über ein Federelement 17 vorgespannt, das sich proximalseitig am proximalen Ende des Schaftes 2 abstützt und distalseitig am proximalen Ende des Zug-/Druckelements 9 anliegt. Über dieses vorzugsweise als Druckfeder ausgebildete Federelement 17 werden das Zug-/Druckelement 9 und das Betätigungselement 7 nach distal gedrückt, wodurch die verschwenkbaren Maulteile 3 des Werkzeugs 4 in die geöffnete Stellung überführt werden und die Griffteile 5 der Handhabe 6 über die in den Führungsbahnen der Griffteile 5 gelagerten Steuerzapfen 13 in die auseinandergespreizte Stellung überführt werden.

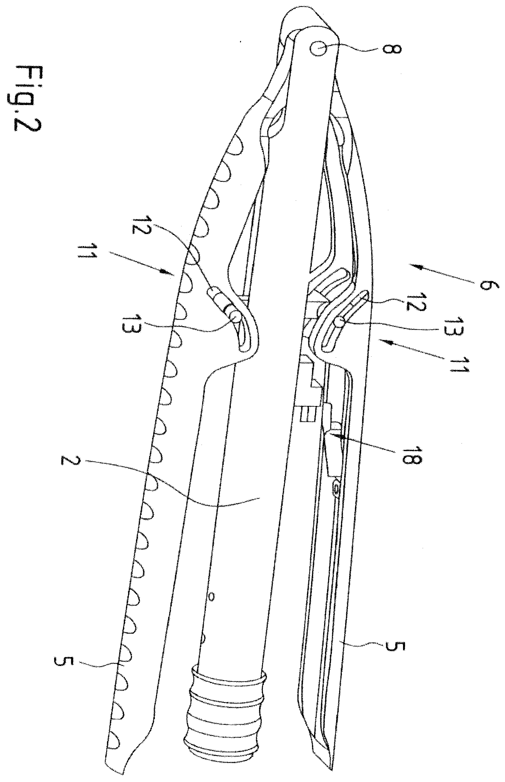

Um den Operateur zu entlasten, sind, wie aus Fig. 2 ersichtlich, die Griffteile 5 der Handhabe 6 in der zusammengedrückten Stellung, also bei geschlossenen Maulteilen 3 des Werkzeugs 4 über einen Arretiermechanismus 18 fixierbar, so dass der Operateur die Griffteile 5 der Handhabe 6 nicht dauerhaft per Handkraft zusammen drücken muss.

Ein solchermaßen ausgebildetes medizinisches Instrument 1 zeichnet sich durch die Ausbildung der Kopplung zwischen den Griffteilen 5 der Handhabe 6 und dem Betätigungselement 7 als Zapfen-Schlitz-Steuerung 11 aus, so dass bei einfachem Aufbau eine direkte, präzise und im Wesentlichen spielfreie Steuerung der Maulteile 3 des Werkzeugs 4 gewährleistet wird.

**Bezugszeichenliste**

| | | | |
|---|---|---|---|
| 1 | medizinisches Instrument | 18 | Arretiermechanismus |
| 2 | Schaft | 19 | Aufnahmevorrichtung |
| 3 | Maulteil | 20 | Verbindungselement |
| 4 | Werkzeug | 21 | Verriegelungselement |
| 5 | Griffteil | 22 | kleine Aufnahme |
| 6 | Handhabe | 23 | große Aufnahme |
| 7 | Betätigungselement | 24 | Bohrung |
| 8 | Schwenkachse | | |
| 9 | Zug-/Druckelement | | |
| 10 | Kopplungsmechanismus | | |
| 11 | Zapfen-Schlitz-Steuerung | | |
| 12 | Führungsbahn | | |
| 13 | Steuerzapfen | | |
| 14 | Längsachse | | |
| 15 | Grundkörper | | |
| 16 | Arm | | |
| 17 | Federelement | | |

## Patentansprüche

1. Medizinisches Instrument mit einem hohlen Schaft (2), an dessen proximalem Ende eine aus mindestens zwei Griffteilen (5) bestehende Handhabe (6) und an dessen distalem Ende ein Werkzeug (4) angeordnet ist, wobei das Werkzeug (4) mittels der Handhabe (6) über ein in dem Schaft (2) axial verschiebbar gelagertes Betätigungselement (7) betätigbar ist, wobei das Betätigungselement (7) ein Zug-/Druckelement (9) aufweist, das als axialverschiebbar im Schaft (2) gelagertes kreuzförmiges Element mit einem in Instrumentenlängsachse (14) ausgerichteten Grundkörper (15) und wenigstens zwei nach außen abstehenden, stabförmigen Armen (16) ausgebildet ist, wobei
die Griffteile (5) der Handhabe (6) und die Arme (16) des Zug-/ Druckelements (9) über eine Zapfen-Schlitz-Steuerung (11) in Wirkverbindung miteinander stehen, und wobei
das Betätigungselement (7) in Instrumentenlängsachse (14) mehrteilig ausgebildet ist, wobei die einzelnen Teile des Betätigungselements (7) über mindestens einen Kopplungsmechanismus (10) miteinander koppelbar sind, **dadurch gekennzeichnet, dass** der Kopplungsmechanismus (10) eine Aufnahmevorrichtung (19) mit mehreren Aufnahmen unterschiedlicher Querschnittsgestalt zur Aufnahme von Verbindungselementen (20) entsprechender Querschnittsgestalt aufweist.

2. Medizinisches Instrument nach Anspruch 1, **dadurch gekennzeichnet, dass** die Aufnahmevorrichtung (19) für die Kopplung von mindestens einem Verbindungselement (20) ein Verriegelungselement (21) aufweist.

3. Medizinisches Instrument nach Anspruch 1, **dadurch gekennzeichnet, dass** die stabförmigen Arme (16) spiegelsymmetrisch am Grundkörper (15) angeordnet und ausgebildet sind.

4. Medizinisches Instrument nach einem der vorangehenden Ansprüche, **dadurch gekennzeichnet, dass** die Zapfen-Schlitz-Steuerung (11) aus direkt oder indirekt an einem der miteinander zu koppelnden Bauteile, nämlich den Griffteilen (5) oder den Armen (16), ausgebildeten Führungsbahnen (12) und direkt oder indirekt an dem anderen der miteinander zu koppelnden Bauteilen, nämlich den Armen (16) oder den Griffteilen (5), ausgebildeten und in den Führungsbahnen (12) gelagerten Steuerzapfen (13) besteht.

5. Medizinisches Instrument nach Anspruch 4, **dadurch gekennzeichnet, dass** die Führungsbahnen (12) in den Griffteilen (5) der Handhabe (6) ausgebildet sind und die Steuerzapfen (13) mit den Armen (16) gekoppelt sind.

6. Medizinisches Instrument nach Anspruch 4, **dadurch gekennzeichnet, dass** die Steuerzapfen (13) an den freien Enden der beiden Arme (16) angeordnet sind.

7. Medizinisches Instrument nach Anspruch 6, **dadurch gekennzeichnet, dass** der in Instrumentenlängsachse (14) ausgerichtete Grundkörper (15) und zwei im Wesentlichen rechtwinklig nach außen abstehende Arme (16) T-förmig ausgebildet sind, wobei die Steuerzapfen (13) an den freien Enden der beiden Arme (16) angeordnet sind.

8. Medizinisches Instrument nach Anspruch 1 bis 7, **dadurch gekennzeichnet, dass** das Zug-/ Druckelement (9) das proximale Ende des Betätigungselements (7) bildet.

9. Medizinisches Instrument nach einem der Ansprüche 1 bis 8, **dadurch gekennzeichnet, dass** das Betätigungselement (7) in distaler Richtung über ein Federelement (17), vorzugsweise eine Druckfeder, vorgespannt ist.

10. Medizinisches Instrument nach einem der Ansprüche 4 bis 9, **dadurch gekennzeichnet, dass** die Steuerzapfen (13) um ihre Längsachse drehbeweglich gelagert sind.

## Claims

1. Medical instrument with a hollow shaft (2) which, at its proximal end, has a handle (6) composed of at least two grip parts (5), and, at its distal end, has a tool (4), wherein the tool (4) can be actuated by means of the handle (6) via an actuation element (7) that is mounted axially movably in the shaft (2), wherein the actuation element (7) has a pull/push element (9) which is designed as a cross-shaped element that is mounted axially movably in the shaft (2) and that has a main body (15) oriented in the longitudinal axis (14) of the medical instrument and at least two outwardly protruding rod-shaped arms (16), wherein the grip parts (5) of the handle (6) and the arms (16) of the pull/push element (9) are operatively connected to each other via a pin-and-slot control system (11), and wherein the actuation element (7) is designed in several parts in the longitudinal axis (14) of the instrument, wherein the individual parts of the actuation element (7) can be coupled to each other via at least one coupling mechanism (10), **characterized in that** the coupling mechanism (10) has a receiving device (19) with several receivers of different cross-sectional shape for receiving connection elements (20) of corresponding cross-sectional shape.

2. Medical instrument according to Claim 1, **characterized in that** the receiving device (19) has a locking element (21) for the coupling of at least one connection element (20).

3. Medical instrument according to Claim 1, **characterized in that** the rod-shaped arms (16) are arranged and configured with mirror symmetry on the main body (15).

4. Medical instrument according to one of the preceding claims, **characterized in that** the pin-and-slot control system (11) is composed of guide tracks (12) configured directly or indirectly on one of the components to be coupled to each other, namely the grip parts (5) or the arms (16), and of control pins (13) configured directly or indirectly on the other of the components to be coupled to each other, namely the arms (16) or the grip parts (5), said control pins (13) being mounted in the guide tracks (12).

5. Medical instrument according to Claim 4, **characterized in that** the guide tracks (12) are formed in the grip parts (5) of the handle (6), and the control pins (13) are coupled to the arms (16).

6. Medical instrument according to Claim 4, **characterized in that** the control pins (13) are arranged on the free ends of the two arms (16).

7. Medical instrument according to Claim 6, **characterized in that** the main body (15) oriented in the longitudinal axis (14) of the instrument and two arms (16) protruding outward substantially at right angles are in a T-shaped configuration, wherein the control pins (13) are arranged on the free ends of the two arms (16).

8. Medical instrument according to Claims 1 to 7, **characterized in that** the pull/push element (9) forms the proximal end of the actuation element (7).

9. Medical instrument according to one of Claims 1 to 8, **characterized in that** the actuation element (7) is pretensioned in the distal direction by a spring element (17), preferably a compression spring.

10. Medical instrument according to one of Claims 4 to 9, **characterized in that** the control pins (13) are mounted so as to be rotatable about their longitudinal axis.

## Revendications

1. Instrument médical comprenant une tige creuse (2) au niveau de l'extrémité proximale de laquelle est disposée une prise (6) constituée d'au moins deux parties de préhension (5) et au niveau de l'extrémité distale de laquelle est disposé un outil (4), l'outil (4) pouvant être actionné au moyen de la prise (6) par le biais d'un élément d'actionnement (7) supporté de manière déplaçable axialement dans la tige (2), l'élément d'actionnement (7) présentant un élément de traction/compression (9) qui est réalisé sous la forme d'un élément cruciforme supporté de manière déplaçable axialement dans la tige (2) avec un corps de base (15) orienté dans l'axe longitudinal de l'instrument (14) et au moins deux bras (16) en forme de barre saillant vers l'extérieur,
les parties de préhension (5) de la prise (6) et les bras (16) de l'élément de traction/compression (9) étant en liaison fonctionnelle les uns avec les autres par le biais d'une commande à tourillon-fente (11), et
l'élément d'actionnement (7) étant réalisé en plusieurs parties dans l'axe longitudinal de l'instrument (14), les parties individuelles de l'élément d'actionnement (7) pouvant être accouplées les unes aux autres par le biais d'au moins un mécanisme d'accouplement (10), **caractérisé en ce que** le mécanisme d'accouplement (10) présente un dispositif de réception (19) avec plusieurs logements de formes différentes en section transversale pour recevoir des éléments de connexion (20) de forme correspondante en section transversale.

2. Instrument médical selon la revendication 1, **caractérisé en ce que** le dispositif de réception (19) pour l'accouplement d'au moins un élément de connexion (20) présente un élément de verrouillage (21).

3. Instrument médical selon la revendication 1, **caractérisé en ce que** les bras en forme de barre (16) sont réalisés et disposés avec une symétrie spéculaire sur le corps de base (15).

4. Instrument médical selon l'une quelconque des revendications précédentes, **caractérisé en ce que** la commande à tourillon-fente (11) se compose de voies de guidage (12) réalisées directement ou indirectement sur l'un des composants à accoupler les uns aux autres, à savoir les parties de préhension (5) ou les bras (16), et de tourillons de commande (13) réalisés directement ou indirectement sur l'autre des composants à accoupler les uns aux autres, à savoir les bras (16) ou les parties de préhension (5), et supportés dans les voies de guidage (12).

5. Instrument médical selon la revendication 4, **caractérisé en ce que** les voies de guidage (12) sont réalisées dans les parties de préhension (5) de la prise (6) et les tourillons de commande (13) sont accouplés aux bras (16).

6. Instrument médical selon la revendication 4, **caractérisé en ce que** les tourillons de commande (13) sont disposés au niveau des extrémités libres des deux bras (16).

7. Instrument médical selon la revendication 6, **caractérisé en ce que** le corps de base (15) orienté dans l'axe longitudinal de l'instrument (14) et deux bras (16) faisant saillie essentiellement à angle droit vers l'extérieur sont réalisés en forme de T, les tourillons de commande (13) étant disposés au niveau des extrémités libres des deux bras (16).

8. Instrument médical selon les revendications 1 à 7, **caractérisé en ce que** l'élément de traction/compression (9) forme l'extrémité proximale de l'élément d'actionnement (7).

9. Instrument médical selon l'une quelconque des revendications 1 à 8, **caractérisé en ce que** l'élément d'actionnement (7) est précontraint dans la direction distale par le biais d'un élément de ressort (17), de préférence un ressort de compression.

10. Instrument médical selon l'une quelconque des revendications 4 à 9, **caractérisé en ce que** les tourillons de commande (13) sont supportés de manière déplaçable en rotation autour de leur axe longitudinal.
